# EUROPÄISCHE PATENTSCHRIFT

(11) **EP 2 377 564 B1**
(45) Veröffentlichungstag und Bekanntmachung des Hinweises auf die Patenterteilung: **17.08.2016**
(21) Anmeldenummer: 11004023.5
(22) Anmeldetag: 08.12.2006
(51) Int. Cl.: A61M 1/36, A61M 5/14, A61M 39/28

(54) **Vorrichtung zur Überwachung eines Zuganges zu einem Patienten, insbesondere eines Gefäßzuganges bei einer extrakorporalen Blutbehandlung**
Device for monitoring access to a patient, in particular vascular access in extracorporeal blood treatment
Dispositif de surveillance d'un accès à un patient, notamment d'un accès aux vaisseaux dans un traitement sanguin extracorporel

(30) Priorität: 11.03.2006 DE 102006011313
(43) Veröffentlichungstag der Anmeldung: 19.10.2011
(62) Teilanmeldung aus: 06829419.8
(73) Patentinhaber: Fresenius Medical Care Deutschland GmbH, 61352 Bad Homburg v.d.H. (DE)
(72) Erfinder: Kopperschmidt, Pascal, Dr., 97456 Dittelbrunn (DE)
(74) Vertreter: Oppermann, Frank

(56) Entgegenhaltungen:
- WO-A-2004/020038
- DE-A1- 19 959 965
- FR-A- 1 306 369
- GB-A- 2 046 095
- US-A1- 4 406 042
- US-A1- 5 309 604
- US-A1- 6 113 577
- US-A1- 2001 007 930

## Beschreibung

Die Erfindung bezieht sich auf einen Punktionsflügel, insbesondere zur Überwachung des Gefäßzuganges bei einer extrakorporalen Blutbehandlung, bei der Blut eines Patienten über eine venöse Schlauchleitung, die eine venöse Punktionskanüle oder -nadel aufweist, dem Patienten zugeführt und über eine arterielle Schlauchleitung, die eine arterielle Punktionskanüle oder -nadel aufweist, von dem Patienten abgeführt wird.

Auf dem Gebiet der Medizintechnik sind eine Vielzahl von Einrichtungen bekannt, mit denen über eine Schlauchleitung einem Patienten Flüssigkeiten zugeführt oder Flüssigkeiten von dem Patienten abgeführt werden können. Dabei erfolgt der Zugang zu dem Patienten im allgemeinen mit einem Katheter zum Einführen in Körperorgane oder einer Kanüle oder Nadel zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Ein Anwendungsfall mit besonders hohen Anforderungen an die Sicherheit des Gefäßzuganges stellt die extrakorporale Blutbehandlung dar, bei der über eine arterielle Schlauchleitung, die eine arterielle Punktionskanüle aufweist, Blut von dem Patienten abgeführt, das Blut durch einen Dialysator geleitet wird und über eine venöse Blutleitung, die eine venöse Punktionskanüle aufweist, dem Patienten wieder zugeführt wird. Dabei besteht trotz regelmäßiger Überwachung des Patientenzugangs durch das Krankenhauspersonal grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, das aufgrund einer maschinenseitigen Luftdetektion zu einem visuellen und/oder optischen Alarm und zur Unterbrechung der Behandlung führt, ist das Herausrutschen der venösen Kanüle und der damit gefürchtete Freifluss des Bluts in die Umgebung nicht ohne weiteres zu detektieren. Wenn das Herausrutschen der venösen Kanüle aber nicht sofort erkannt wird, kann der Patient verbluten.

Zur Lösung dieses Problems sind im Stand der Technik eine Vielzahl unterschiedlicher Vorrichtungen bekannt. Einige dieser Vorrichtungen greifen auf standardmäßig in den Blutbehandlungsmaschinen vorhandene Sicherheitsvorrichtungen zurück und lösen bei einem nicht ordnungsgemäßen Gefäßzugang eine sofortige Unterbrechung des extrakorporalen Blutkreislaufs aus. Die standardmäßig in den Behandlungsmaschinen vorhandenen Sicherheitsvorrichtungen basieren im allgemeinen auf einer Überwachung des Drucks im extrakorporalen Blutkreislauf. In der Praxis hat sich jedoch gezeigt, dass allein mit einer Überwachung des Drucks im extrakorporalen Blutkreislauf das Herausrutschen insbesondere der venösen Punktionskanüle nicht mit der ausreichenden Sicherheit erkannt werden kann. Einige bekannte Sicherheitsvorrichtungen haben zwar eine ausreichende Sensitivität, sie reagieren aber sehr empfindlich auf Lageänderungen des Patienten, was oft zu Fehlalarmen führt. Nachteilig ist auch, das sich die bestehenden Blutbehandlungsvorrichtungen nicht ohne weiteres mit den bekannten Überwachungsvorrichtungen nachrüsten lassen, sondern die Nachrüstung einen aufwendigen und kostenintensiven Eingriff in die Behandlungsmaschinen erfordert.

Die DE 44 32 348 C2 beschreibt eine Sicherheitsvorrichtung für eine blut-, wundsekret- oder infusionsführende Schlauchleitung, die auf eine relative Positionsänderung der Schlauchleitung reagiert. Die bekannte Sicherheitseinrichtung verfügt über einen Magneten, der an der Schlauchleitung befestigt ist, und einen Reedkontakt, der an dem Patienten angebracht wird. Wenn an der Schlauchleitung gezogen wird, ändert sich der Abstand zwischen Magnet und Reedkontakt, so dass ein Alarm ausgelöst wird.

Aus der DE 199 53 068 A1 ist eine mechanische Sicherheitsvorrichtung bekannt, die beispielsweise an der Blutleitung einer Dialysemaschine befestigt werden kann. Die bekannte Sicherheitsvorrichtung verfügt über elastisch vorgespannte Klemmbacken, die von einem am Körper des Patienten fixierten Sperrriegel in der geöffneten Position gehalten werden, wobei die Blutleitung zwischen die Klemmbacken eingelegt wird. Eine Lageänderung der Blutleitung führt zu einem Abreißen des Sperrriegels, so dass die Klemmbacken den Schlauch abklemmen. Dies führt zu einem Druckanstieg im Schlauch, der mit den standardmäßig in den bekannten Dialysevorrichtungen vorhandenen Einrichtungen zur Überwachung des Drucks im extrakorporalen Kreislauf detektiert wird. Nachteilig ist, dass die Befestigung der mechanischen Sicherheitsvorrichtung einerseits am Blutschlauch und andererseits am Patienten umständlich ist. Da die Klemmbacken die Schlauchleitung schlagartig abklemmen, ist es nicht möglich, vor der Unterbrechung der Blutbehandlung nur einen Alarm auszulösen. Auch ist aufgrund der auftretenden Kriechvorgänge der Einsatz von kostengünstigen Kunststoffen als Werkstoff bei einer werkseitigen Montage an der Blutleitung problematisch. Grundsätzlich sollten die Klemmbacken nicht unter ständiger Vorspannung stehen, was aber nur dann der Fall ist, wenn die Klemmbacken geschlossen sind. Da der Schlauch bei geschlossenen Klemmbacken aber abgequetscht ist, müssen die Klemmbacken dennoch unter Vorspannung stehen. Wenn die Vorrichtung aus kostengünstigen Kunststoffen gefertigt ist, könnte dies aber in Folge von Kriechvorgängen bereits bei Raumtemperatur mit der Zeit zu einer bleibenden Abnahme der Vorspannung der Klemmbacken und somit zur Unbrauchbarkeit der Vorrichtung führen.

Vorrichtungen zur Fixierung von Schlauchleitungen in Form einer Schlaufe sind beispielsweise aus der US 5,309,604 A1, DE 199 59 965 A1, US 4, 406,042 A1 und WO 2004/020038 A1 bekannt. Die Vorrichtungen erlauben, Schlauchleitungen für medizinische Einrichtungen in der erforderlichen Länge zu fixieren.

Die DE 199 59 965 A1 und US 4, 406,042 A1 beschreiben Clips, mit denen die Schlauchleitung in Form einer Schlaufe fixiert werden können. Dabei werden die betreffenden Schlauchleitungsabschnitte fest von dem Clip umfasst, so dass sich die Schlaufe nicht zusammenziehen kann. Die Clips sind der Schlauchleitung lose beigefügt. Die US 5,309,604 A1 schlägt einen Befestigungsclip vor, der eine ringförmige Öse aufweist, die den einen Schlauchleitungsabschnitt umschließt, und eine Klammer aufweist, in die der andere Schlauchleitungsabschnitt eingelegt werden kann.

Aus der WO 2004/020038 A1 ist eine Vorrichtung zur subkutanen Verabreichung von Medikamenten bekannt, die eine auf die Haut des Patienten aufzulegende Medikamentenkammer umfasst, die eine Kanüle aufweist. An der Medikamentenkammer ist eine Schlauchleitung angeschlossen. Um die Schlauchleitung auf die erforderliche Länge einstellen zu können, verfügt die bekannte Vorrichtung über ein separates Befestigungsteil mit zwei Führungen, in denen die betreffenden Schlauchleitungsabschnitte, die eine Schlaufe bilden, geführt sind.

Die US 6 113 577 A1 beschreibt eine Vorrichtung zur Fixierung eines Punktionsflugels am Arm des Patienten, die ein Herausrutschen der Kanüle aus der Punktionsstelle verhindern soll. Die Vorrichtung verfügt über eine Halterung, in die der Punktionsflügel passend eingesetzt wird, wobei der Punktionsflügel in der Halterung mit seitlichen Stegen fixiert wird. Die Halterung für den Punktionsflügel wird mit einem Halteband am Arm des Patienten befestigt. An dem Halteband befindet sich eine Halteklammer, mit der auch die an dem Punktionsflügel angeschlossene Schlauchleitung am Arm des Patienten fixiert wird.

Der Erfindung liegt die Aufgabe zugrunde, eine einfach zu handhabende, kostengünstig herstellbare und jederzeit nachrüstbare Vorrichtung zu schaffen, die eine sichere Überwachung eines Patientenzuganges erlaubt.

Die Lösung dieser Aufgaben erfolgt erfindungsgemäß mit den Merkmalen der Patentanspruchs 1. Vorteilhafte Ausführungsformen der Erfindung sind Gegenstand der Unteransprüche.

Der erfindungsgemäße Punktionsflügel zur Überwachung eines Patientenzuganges beruht darauf, dass eine Schlaufe in der flüssigkeitsführenden Schlauchleitung gebildet wird. Es wird davon ausgegangen, dass ein Herausrutschen der Punktionskanüle oder des Katheters auf das Angreifen von Zugkräften an der Schlauchleitung zurückzuführen ist. Wenn die Schlauchleitung auch Zug beansprucht wird, zieht sich die Schlaufe zwangsläufig zu. Dies führt zu einem erhöhten Druckverlust in der Schlauchleitung, der leicht zu detektieren ist.

Die bekannten Blutbehandlungsvorrichtungen verfügen bereits über eine Einrichtung zur Überwachung des Drucks im extrakorporalen Blutkreislauf. Wenn der Druck vorgegebene Grenzen überschreitet, kann die Blutbehandlungsvorrichtung einen Alarm geben und/oder die Blutbehandlung unterbrechen. Die hierzu erforderlichen mechanischen Einrichtungen sind in den bekannten Blutbehandlungsvorrichtungen vorhanden. Daher ist es lediglich erforderlich, die vorgegebenen Grenzwerte für den Druck im extrakorporalen Blutkreislauf auf die verwendete Schlauchleitung abzustimmen.

Von Vorteil ist, dass nicht nur ein fehlerhafter Patientenzugang leicht erkannt werden kann, sondern dass die Bildung einer Schlaufe gleichsam die Übertragung von Zugkräften auf den Katheter oder die Kanüle schwächen kann. Wenn der Schlauch auf Zug beansprucht wird, zieht sich zunächst die Schlaufe zu, so dass die Zugkräfte nicht sofort auf den Katheter oder die Kanüle übertragen werden. Erst wenn sich die Schlaufe so weit zugezogen hat, dass der Schlauch abknickt, werden die Zugkräfte auf den Katheter oder die Kanüle übertragen. Dann setzt aber schon der Schutzmechanismus ein. Es reicht bereits ein leichter Knick aus, um eine Erhöhung des Druckverlusts in der Schlauchleitung sicher detektieren zu können. Außerdem ist vorteilhaft, dass sich der Staudruck mit zunehmender Strömungsgeschwindigkeit der Flüssigkeit schneller aufbaut. Dies ist insbesondere dann der Fall, wenn die Schlauchleitung aus einem flexiblen Material besteht, das leicht nachgibt.

Wenn die Schlauchlänge entsprechend großzügig bemessen und dem Patienten ein gewisser Bewegungsfreiraum gegeben wird, entstehen nur wenig Fehlalarme, weil im Rahmen üblicher Bewegungen keine oder nur geringe Zugkräfte am Blutschlauch auftreten können. Zudem ist es möglich, die Einrichtung zum Zu- oder Abführen der Flüssigkeit bei einem Störfall nicht sofort abzuschalten, sondern nur einen Alarm auszulösen. Sollte Alarm ausgelöst werden, kann das Krankenhauspersonal den Knick durch Zurückführen der Schlauchleitung in die Schlaufenform beseitigen, ohne dass die Behandlung unterbrochen werden muss.

Es hat sich als vorteilhaft erwiesen, wenn die Schlaufe unmittelbar hinter der Punktionskanüle oder dem Katheter gebildet wird, da die Schlauchleitung an dieser Stelle im Allgemeinen geringeren Beanspruchungen in Form von Lageänderungen des Patienten ausgesetzt ist.

Der erfindungsgemäße Punktionsflügel (Katheter) weist ein Befestigungsmittel zum lösbaren Befestigen eines Schlauchsegments einer Schlauchleitung auf, die an der Punktionskanüle entweder angeschlossen oder anschließbar ist. Die Schlaufe kann einfach dadurch gebildet werden, dass das

Schlauchsegment hinter der Punktionskanüle zu einer Schleife geformt und das hinter der Schleife liegende Schlauchsegment mit den Befestigungsmitteln fixiert wird. Wenn die Schlauchleitung in dem Befestigungsmittel lose geführt ist, kann sich die Schlaufe bei einer Zugbeanspruchung zunächst so lange zuziehen, bis die Schlauchleitung letztlich abknickt.

Vorzugsweise ist das Befestigungsmittel derart ausgebildet, dass das Schlauchsegment lösbar befestigt werden kann. Es ist aber grundsätzlich auch möglich, dass hinter der Punktionskanüle die Schlaufe nicht von Hand gebildet wird, sondern die Schlauchleitung an dem Kathether (Punktionsflügel) dauerhaft eine Schlaufe bildet.

Die Befestigungsmittel für das Schlauchsegment weisen vorzugsweise eine Öse auf, durch die das Schlauchsegment geführt werden kann, so dass sich die Schlaufe bei Zugbeanspruchung zusammenziehen kann. Vorzugsweise ist die Öse zum Einlegen der Schlauchleitung aufspreizbar. Sie kann beispielsweise als Clip oder dgl. für die Schlauchleitung ausgebildet sein.

Im Folgenden werden verschiedene Ausführungsbeispiele der Erfindung unter Bezugnahme auf die Zeichnungen näher erläutert.

Es zeigen:
- Fig. 1: die wesentlichen Komponenten einer Hämodialysevorrichtung zusammen mit der erfindungsgemäßen Vorrichtung zur Überwachung des Patientenzuganges in stark vereinfachter schematischer Darstellung,
- Fig. 2: ein erstes Ausführungsbeispiel der Vorrichtung in perspektivischer Darstellung, die aber nicht Gegenstand der Erfindung ist,
- Fig. 3: die Vorrichtung von Fig. 2 zusammen mit einer Punktionskanüle und einer Schlauchleitung, wobei die Schlauchleitung nicht auf Zug beansprucht worden ist,
- Fig. 4: die Vorrichtung von Fig. 2, wobei die Schlauchleitung auf Zug beansprucht worden ist,
- Fig. 5: ein zweites Ausführungsbeispiel der Vorrichtung in perspektivischer Darstellung, die aber nicht Gegenstand der Erfindung ist,
- Fig. 6: ein Ausführungsbeispiel des erfindungsgemäßen Punktionsflügels in perspektivischer Darstellung,
- Fig. 7: der Punktionsflügel von Fig. 6 zusammen mit einer Schlauchleitung in perspektivischer Darstellung und
- Fig. 8: eine weitere Ansicht des Punktionsflügels von Fig. 6 in perspektivischer Darstellung.

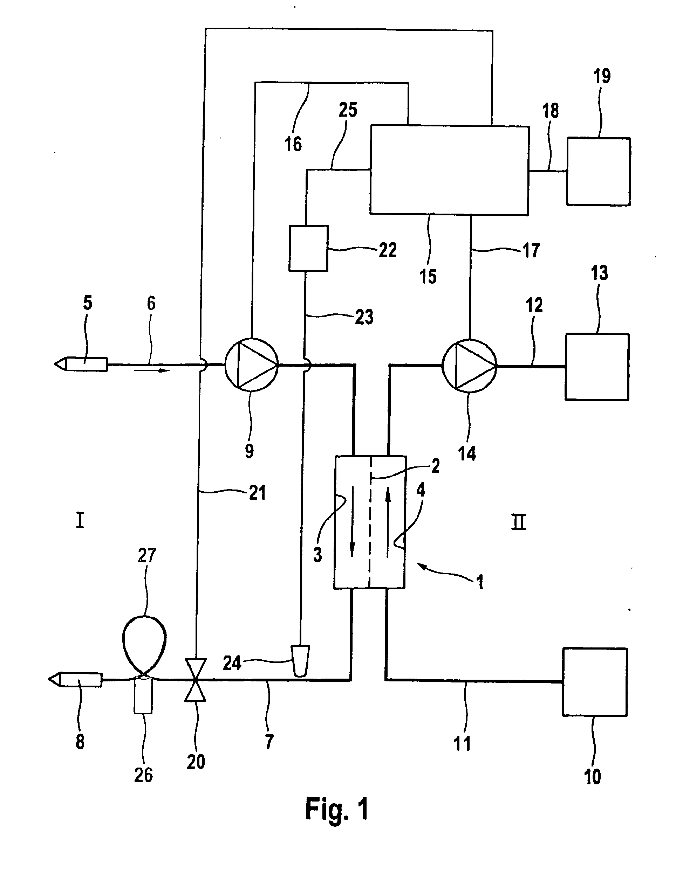

Fig. 1 zeigt die wesentlichen Komponenten einer Hämodialysevorrichtung, die über eine Vorrichtung zur Überwachung des venösen Gefäßzugangs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist. An einer Arterie des Patienten ist mittels einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators 1 geht eine venöse Schlauchleitung 7 ab, die mittels einer venösen Punktionskanüle 8 an einer Vene des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysierflüssigkeitskreislauf II der Hämodialysevorrichtung umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschlossen wird, wenn die venöse Punktionskanüle (Nadel) aus dem Gefäßzugang heraus rutschen sollte. Darüber hinaus stoppt die Steuereinheit 15 nach dem Herausrutschen der Kanüle die Blutpumpe 9.

Zur Überwachung des Drucks in der arteriellen Schlauchleitung weist die Dialysevorrichtung eine Überwachungseinrichtung 22 auf, die über eine Datenleitung 23 mit einem Drucksensor 24 verbunden ist, der den Druck in der venösen Schlauchleitung 7 misst. Die Drucküberwachungseinrichtung 22 kommuniziert mit der zentralen Steuereinheit 15 über eine weitere Datenleitung 25.

Die Vorrichtung zur Überwachung des venösen Gefäßzugangs weist nachfolgend noch im Einzelnen beschriebene Mittel 26 zum Fixieren eines Schlauchsegments der venösen Schlauchleitung 7 in Form einer Schlaufe 27 auf. Die Schlaufe wird stromauf der venösen Punktionskanüle 8 vorzugsweise an einem Abschnitt der Schlauchleitung gebildet, in dem die Schlauchleitung noch am Körper des Patienten, beispielsweise an dessen Unterarm, anliegt.

Während der Dialysebehandlung wird die venöse Schlauchleitung 7 nicht auf Zug beansprucht. Die Drucküberwachungseinrichtung 22 misst einen Druck P in der venösen Schlauchleitung, der innerhalb vorgegebener Grenzen liegt. Die typischen venösen Drücke liegen bei etwa 100 bis 200 mmHg. Es sei angenommen, dass die Druckmesseinrichtung 22 einen venösen Druck von 150 mmHg misst. Bei derartigen Druckverhältnissen wird ein Grenzwertfenster mit einer Breite von 100 mmHg definiert, wobei der untere Grenzwert für den Druck bei 100 mmHg und der obere Grenzwert bei 200 mmHg liegt. Liegt der gemessene Druck ober- bzw. unterhalb der vorgegebenen Grenzen von 100 bzw. 200 mmHg, löst die zentrale Steuereinheit 15 einen optischen und/oder akustischen Alarm aus. Wenn an der venösen Schlauchleitung 7 stromauf der Schlaufe 27 gezogen wird, besteht die Gefahr, dass die Punktionskanüle 8 aus der Vene des Patienten herausrutscht. Bleibt dies unbemerkt, besteht für den Patienten Lebensgefahr. Der Druckabfall in der venösen Schlauchleitung durch Verlust des Innendrucks im Patientenzugang von etwa 15 bis 25 mmHg kann aber nicht zu einer Unterschreitung des unteren Grenzwertes für den venösen Druck von 100 mmHg führen, so dass ohne die erfindungsgemäße Überwachungsvorrichtung das Herausrutschen der venösen Punktionskanüle nicht erkannt wird.

Da die Schlauchleitung aber oberhalb der Punktionskanüle eine Schlaufe bildet, führt das Herausrutschen der Punktionskanüle zu der Auslösung eines Alarms und/oder die Unterbrechung der Blutbehandlung. Wenn an der venösen Schlauchleitung 7 gezogen wird, zieht sich die Schlaufe 27 zunächst zusammen, so dass sich die Zugspannung zunächst "abfedern" lässt. Eine weitere Zugbeanspruchung führt jedoch dazu, dass sich die Schlaufe 27 so lange zusammenzieht, bis der Schlauch letztlich abknickt. Da sich das Blut an der Knickstelle staut, steigt der venöse Druck in der Schlauchleitung stromauf der Knickstelle. Der Staudruck hängt von dem verbleibenden offenen Querschnitt des abgeknickten Schlauchsegments an der Knickstelle und vom Blutfluss ab. Die Druckmesseinrichtung 22 misst mit dem stromauf der Schlaufe 27 angeordneten Drucksensor 24 den Staudruck vor der Knickstelle. Innerhalb kurzer Zeit, ca. 1 bis 2 Sekunden, steigt der Druck von 150 mmHg aufgrund der Verengung an der Knickstelle stark an, wodurch der obere Grenzwert von 200 mmHg für den venösen Druck überschritten wird. Folglich löst die zentrale Steuereinheit 15 einen Alarm aus und unterbricht die Blutbehandlung durch Schließen der venösen Schlauchklemme 20 und Stoppen der Blutpumpe 9.

Für die Überwachung können verschiedene Grenzwerte unterschiedlicher Höhe definiert werden, so dass darauf geschlossen werden kann, ob die Punktionskanüle aus dem Gefäßzugang herauszurutschen droht oder teilweise oder ganz herausgerutscht ist. Bei Überschreiten der einzelnen Grenzwerte können unterschiedliche Voralarme oder Alarme ausgelöst werden.

Da eine nennenswerte Druckänderung beim Zuziehen der Schlaufe erst relativ spät, d.h. erst mit der Knickbildung auftritt, muss sichergestellt werden, dass die Schlauchleitung tatsächlich abknickt. Es ist vorteilhaft, dies durch eine konstruktive oder strukturelle Anisotropie der Schlauchleitung zu unterstützen, wobei die Form und/oder Beschaffenheit des Materials des Schlauches an der Knickstelle abweichend von der Form und/oder Beschaffenheit des Materials außerhalb der Knickstelle verändert wird. So kann beispielsweise eine Sollknickstelle dadurch geschaffen werden, dass die Schlauchwandung dünner ausgebildet wird oder beispielsweise von der runden Querschnittsform abweicht. So kann die runde Schlauchleitung an der Knickstelle beispielsweise einen elliptischen Querschnitt aufweisen.

Nachfolgend wird ein erstes Ausführungsbeispiel der Mittel beschrieben, mit denen sich auf einfache Weise eine Schlaufe in der Schlauchleitung bilden lässt, wobei diese Fixierungsmittel aber nicht Gegenstand der Erfindung sind. Die Fixierungsmittel können jederzeit an die bestehende Schlauchleitung einer konventionellen Dialysemaschine angebracht werden, die im Allgemeinen bereits über eine Drucküberwachungseinrichtung verfügt, die beim Über- und/oder Unterschreiten von vorgegebenen Grenzwerten reagiert.

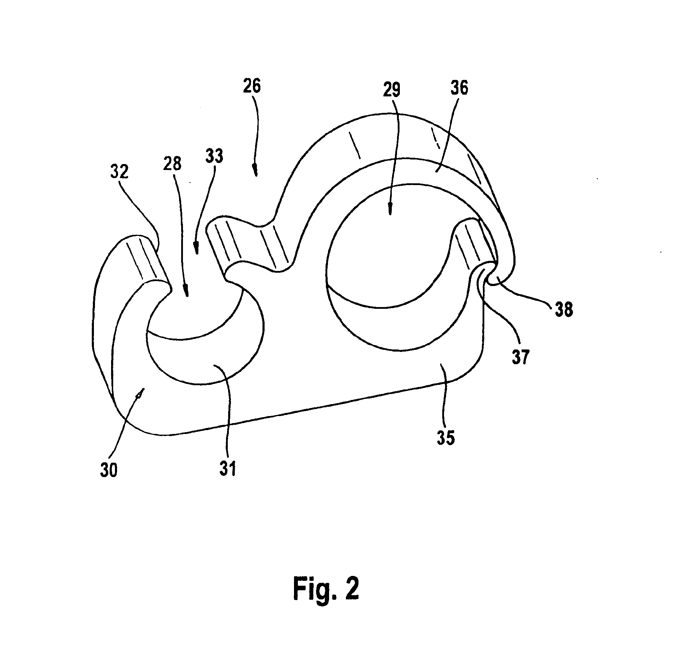

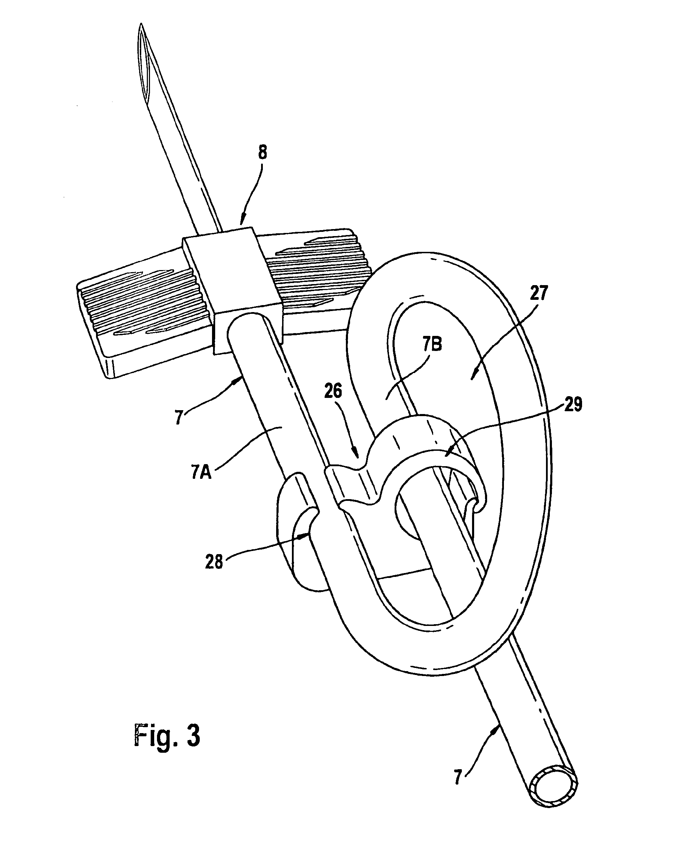

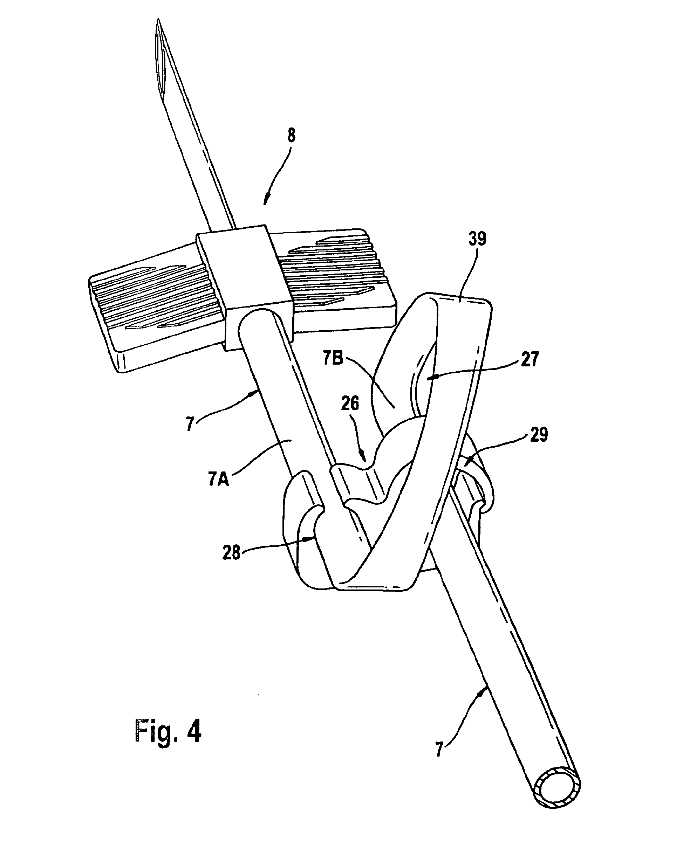

Fig. 2 zeigt die Fixierungsmittel 26 in perspektivischer Darstellung ohne venöse Schlauchleitung, während die Figuren 3 und 4 die Fixierungsmittel 26 zusammen mit der venösen Schlauchleitung zeigen, wobei die Schlauchleitung in Fig. 3 nicht und die Leitung in Fig. 4 auf Zug beansprucht worden ist.

Die Fixierungsmittel 26 verfügen über zwei Befestigungsmittel 28, 29 für die venöse Schlauchleitung 7. Die beiden Befestigungsmittel 28, 29 sind derart angeordnet, dass die damit gehaltenen Schlauchsegmente der Schlauchleitung parallel zueinander verlaufen. Mit dem ersten Befestigungsmittel 28 werden die Fixierungsmittel 26 an einem Schlauchsegment 7A der venösen Schlauchleitung 7, das stromauf der venösen Punktionskanüle 8 liegt, vorzugsweise unmittelbar vor der Punktionskanüle, angebracht. Das erste Befestigungsmittel 28 ist ein Kunststoff-Spritzgussteil, das in der Art einer Klammer 30 ausgebildet ist, in die das Schlauchsegment 7A eingelegt werden kann. Die Klammer 30 weist eine zentrale Öffnung 31 auf, deren Durchmesser geringfügig kleiner als der Durchmesser des Schlauchsegments 7A ist, so dass das Schlauchsegment von der Klammer klemmend gehalten wird. Die Klammer 30 umschließt das Schlauchsegment 7A bis auf einen oberen Spalt 33, durch den das Schlauchsegment eingeschoben wird, wodurch sich die Klammer leicht aufspreizt.

Zur Befestigung der Fixierungsmittel an der Schlauchleitung können aber anstelle einer Klammer auch zwei parallele Stege vorgesehen sein, deren Abstand geringfügig kleiner als der Durchmesser der Schlauchleitung, so dass die Schlauchleitung zwischen den Stegen leicht klemmend fixiert wird.

An das erste Befestigungsmittel 28 ist ein zweites Befestigungsmittel 29 angeformt, das ein anderes Schlauchsegment 7B der venösen Schlauchleitung 7 hält, wenn die Schlauchleitung eine Schlaufe 27 bildet. Das zweite Befestigungsmittel 29 ist als Öse ausgebildet, die geöffnet oder verschlossen werden kann. Die Öse 29 ist ein Kunststoff-Spritzgussteil mit einem unteren bogenförmigen Teil 35 und einem oberen bogenförmigen Teil 36. Das untere und obere bogenförmige Teil 35, 36 weisen jeweils einen Verschlusshaken 37, 38 auf, die beim Zusammendrücken des unteren und oberen Teils 35, 36 einschnappend ineinander greifen.

Zum Einlegen des Schlauchsegments7B wird das obere bogenförmige Teil 36 nach oben gebogen, so dass das Schlauchsegment in den Spalt zwischen dem oberen und unteren Teil 35, 36 in die Öse 29 eingelegt werden kann. Dann wird die Öse durch Zusammendrücken des oberen und unteren Teils verschlossen, so dass das Schlauchsegment 7B in der Öse unverlierbar gesichert ist. Das Schlauchsegment liegt dabei lose in der Öse, so dass sich die Schlaufe beim Ziehen an der Schlauchleitung zusammenziehen kann. Wenn an der Schlauchleitung 7 gezogen wird, zieht sich die Schlaufe zusammen, bis die Schlauchleitung einknickt. Die Knickstelle ist in Fig. 4 mit dem Bezugszeichen 39 versehen.

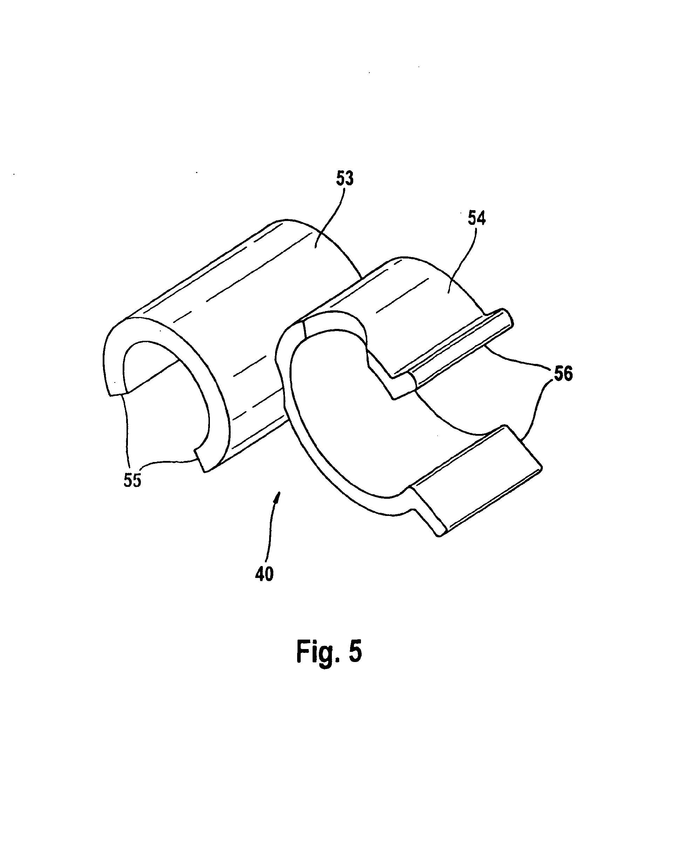

Fig. 5 zeigt in perspektivischer Darstellung ein weiteres Ausführungsbeispiel der Fixierungsmittel 40, die aber ebenfalls nicht Gegenstand der Erfindung sind. Die Fixierungsmittel weisen zwei miteinander verbundene Ösen 53, 54 auf, von denen die eine das erste Schlauchsegment 7A und die andere das zweite Schlauchsegment 7B aufnimmt. Beide Ösen können nicht verschlossen werden, sondern halten das jeweilige Schlauchsegment leicht klemmend fest. Während die Endstücke 55 der einen Öse 53 nach innen weisen, zeigen die Endstücke 56 der anderen Öse 54 nach außen. Es können aber auch die Endstücke beider Ösen nach innen bzw. außen zeigen. Die nach außen weisenden Endstücke haben den Vorteil, dass sich die Öse leichter aufspreizen und die Schlauchleitung leichter einlegen lässt.

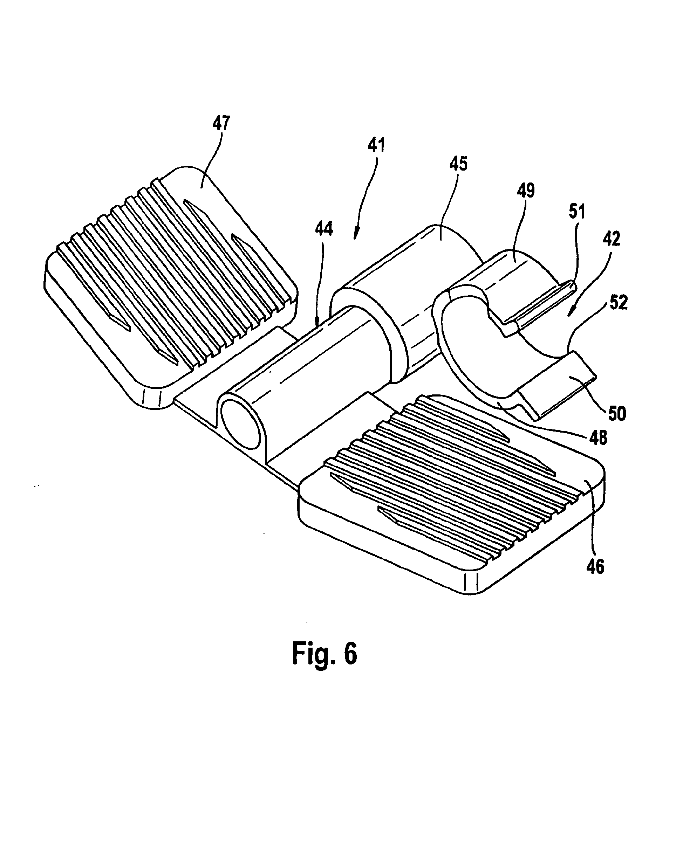

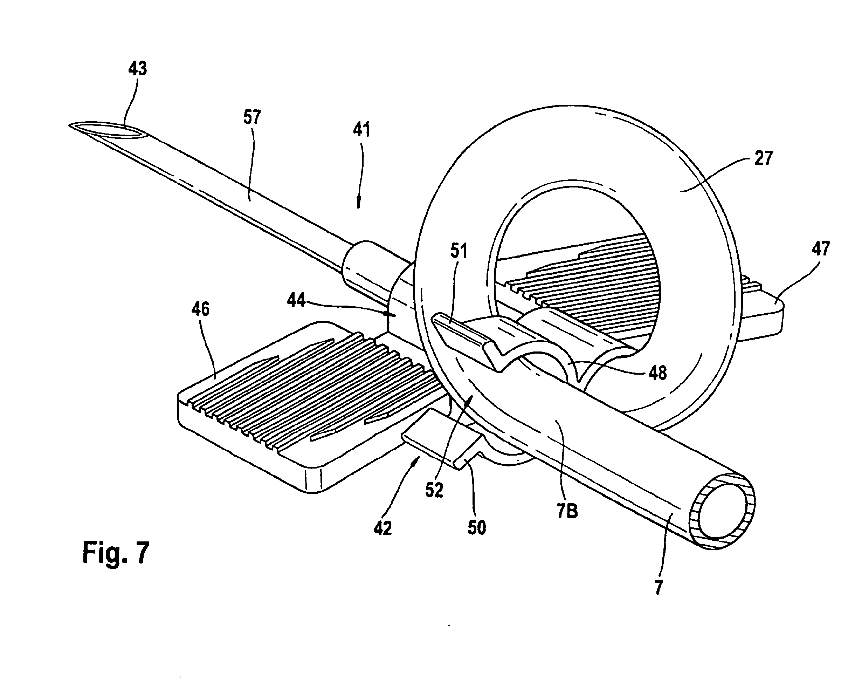

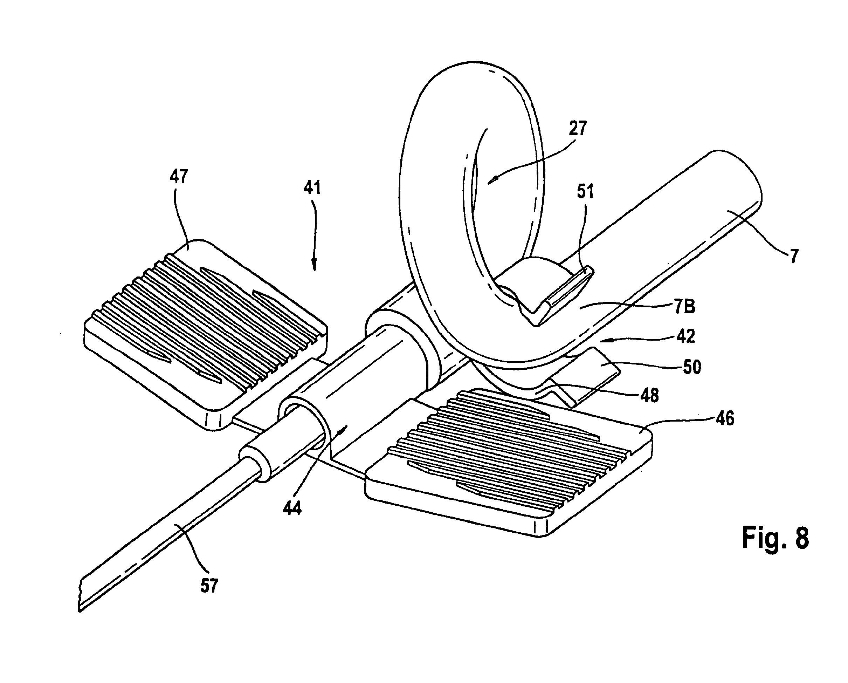

Die Figuren 6 bis 8 zeigen perspektivische Darstellungen aus unterschiedlichen Richtungen einer Ausführungsform der erfindungsgemäßen Fixierungsmittel 41. Diese Ausführungsform unterscheidet sich von dem unter Bezugnahme auf die Figuren 2 bis 5 beschriebenen Ausführungsbeispielen dadurch, dass die Fixierungsmittel als Punktionsflügel 41 ausgebildet sind. Der erfindungsgemäße Punktionsflügel unterscheidet sich von einer konventionellen Drehflügelkanüle dadurch, dass der Punktionsflügel über ein Befestigungsmittel 42 zur lösbaren Befestigung eines Schlauchsegments 7B einer an der Kanüle angeschlossenen oder anschließbaren Schlauchleitung 7 verfügt. Die Punktionsflügel 41 weist eine Kanülennadel 57 mit einer Anschlifföffnung 43 und ein Schlauchanschlussstück 44 auf, mit dem die Schlauchleitung 7 fest verbunden, beispielsweise verklebt oder verschweißt ist. Zum Anschluss der Schlauchleitung können das Schlauchanschlussstück 44 und das Schlauchleitungsende aber auch mit geeigneten Konnektoren versehen sein. In Fig. 6 ist die Punktionskanüle, die in das Schlauchanschlussstück eingesetzt ist, nicht dargestellt.

Das Schlauchanschlussstück 44 weist einen zylindrischen Körper 45 zur Aufnahme des Schlauchendstücks auf, an dem zu beiden Seiten jeweils ein Flügel 46, 47 angeformt ist. Mit dem zylindrische Körper 45 des Schlauchanschlussstücks 44 ist das Befestigungsmittel 42 verbunden. Das Befestigungsmittel 42 ist eine aufspreizbare Öse, in die das Schlauchsegment 7B der Schlauchleitung 7 nach Bildung der Schlaufe 27 seitlich eingeschoben werden kann. Die Öse besteht aus zwei bogenförmigen Kunststoffstücken 48, 49, die an das Schlauchanschlussstück seitlich angeformt sind und jeweils in einem abgewinkelten Schenkel 50, 51 enden. Nach Bilden der Schlaufe 27 wird die Schlauchleitung 7 durch den Spalt 52 zwischen den beiden Schenkeln 50, 51 seitlich in die Öse eingelegt, in der die Leitung gegen Herausrutschen gesichert ist. Die Schlauchleitung ist wieder lose in der Öse geführt, so dass sich die Schlaufe unter Zugbeanspruchung zusammenziehen und abknicken kann.

## Patentansprüche

1. Punktionsflügel zur Überwachung eines Zugangs zu einem Patienten für eine Einrichtung, mit der über eine Schlauchleitung eine Flüssigkeit zu dem Patienten geführt oder von dem Patienten abgeführt wird, insbesondere zur Überwachung des Gefäßzuganges bei einer extrakorporalen Blutbehandlung, bei der Blut eines Patienten über eine venöse Schlauchleitung, die eine venöse Punktionskanüle aufweist, dem Patienten zugeführt und über eine arterielle Schlauchleitung, die eine arterielle Punktionskanüle aufweist, von dem Patienten abgefühlt wird, **dadurch gekennzeichnet, dass** der Punktionsflügel ein mit dem Punktionsflügel einstückiges Befestigungsmittel (42) zum lösbaren Befestigen eines Schlauchsegments (7B) einer an der Kanüle angeschlossenen oder anschließbaren Schlauchleitung (7) in Form einer Schlaufe (27) aufweist, so dass sich die Schlaufe bei einer Beanspruchung der Schlauchleitung auf Zug zusammenziehen kann.

2. Punktionsflügel nach Anspruch 1, **dadurch gekennzeichnet, dass** der Punktionsflügel ein Schlauchanschlussstück (44) aufweist, an das die Schlauchleitung (7) angeschlossen oder anschließbar ist, und dass das Befestigungsmittel (42) einstückiger Bestandteil des Schlauchanschlussstücks ist.

3. Punktionsflügel nach Anspruch 1 oder 2, **dadurch gekennzeichnet, dass** das Befestigungsmittel als Öse (42) ausgebildet ist, durch die das Schlauchsegment (7B) der Schlauchleitung (7) geführt werden kann.

4. Punktionsflügel nach Anspruch 3, **dadurch gekennzeichnet, dass** die Öse (42) zum Einlegen des Schlauchsegments (7B) der Schlauchleitung (7) aufspreizbar ist.

5. Punktionsflügel nach einem der Ansprüche 1 bis 4 mit einer Schlauchleitung (7) zum Zuführen einer Flüssigkeit zu einem Patienten oder Abführen einer Flüssigkeit von einem Patienten, **dadurch gekennzeichnet, dass** die Schlauchleitung (7) mit einer Sollknickstelle ausgebildet ist.

6. Punktionsflügel nach Anspruch 5, **dadurch gekennzeichnet, dass** die Wandung der Schlauchleitung (7) im Bereich der Sollknickstelle eine geringere Stärke aufweist.

7. Punktionsflügel nach Anspruch 5, **dadurch gekennzeichnet, dass** die Schlauchleitung (7) mit Ausnahme im Bereich der Sollknickstelle einen runden Querschnitt aufweist, wobei die Schlauchleitung im Bereich der Sollknickstelle einen nicht runden Querschnitt aufweist.

## Claims

1. Butterfly cannula for monitoring an access to a patient for a device, by means of which device a fluid can be supplied to the patient or can be removed from the patient via a hose line, in particular for monitoring the vascular access in an extracorporeal blood treatment, during which blood of a patient is supplied to the patient via a venous hose line that comprises a venous puncture cannula and is removed from the patient via an arterial hose line that comprises an arterial puncture cannula,
**characterised in that** the butterfly cannula comprises an attachment means (42), integral with the butterfly cannula, for releasably attaching a hose segment (7B) of a hose line (7), which is or can be connected to the cannula, in the form of a loop (27) such that the loop can tighten if the hose line is subjected to tensile stress.

2. Butterfly cannula according to claim 1, **characterised in that** the butterfly cannula comprises a hose connector (44) to which the hose line (7) is or can be connected, and **in that** the attachment means (42) is an integral component of the hose connector.

3. Butterfly cannula according to either claim 1 or claim 2, **characterised in that** the attachment means is formed as an eyelet (42) through which the hose segment (7B) of the hose line (7) can be guided.

4. Butterfly cannula according to claim 3, **characterised in that** the eyelet (42) can be spread apart in order to insert the hose segment (7B) of the hose line (7).

5. Butterfly cannula according to any of claims 1 to 4, comprising a hose line (7) for supplying a fluid to a patient or removing a fluid from a patient, **characterised in that** the hose line (7) is formed having a predetermined bending point.

6. Butterfly cannula according to claim 5, **characterised in that** the walls of the hose line (7) are of a lower thickness in the region of the predetermined bending point.

7. Butterfly cannula according to claim 5, **characterised in that**, with the exception of the region of the predetermined bending point, the hose line (7) has a round cross section, the hose line having a non-round cross section in the region of the predetermined bending point.

## Revendications

1. Ailette de ponction servant à surveiller un accès à un patient, destinée à un dispositif, à l'aide duquel un liquide est guidé en direction du patient par l'intermédiaire d'un conduit flexible ou est évacué du patient, en particulier servant à surveiller l'accès vasculaire lors d'un traitement du sang extracorporel, lors duquel le sang d'un patient est amené au patient par l'intermédiaire d'un conduit flexible veineux, qui présente une canule de ponction veineuse, et est évacué du patient par l'intermédiaire d'un conduit flexible artériel, qui présente une canule de ponction artérielle,
**caractérisée en ce que** l'ailette de ponction présente un moyen de fixation (42) d'un seul tenant avec l'ailette de ponction, servant à fixer de manière amovible un segment de flexible (7B) d'un conduit flexible (7) raccordé ou pouvant être raccordé à la canule sous la forme d'une boucle (27) si bien que la boucle peut se contracter lorsque le conduit flexible est soumis à l'action d'une traction.

2. Ailette de ponction selon la revendication 1, **caractérisée en ce que** l'ailette de ponction présente une pièce de raccordement de flexible (44), à laquelle le conduit flexible (7) est raccordé ou peut être raccordé, et **en ce que** le moyen de fixation (42) est un élément constitutif d'un seul tenant de la pièce de raccordement de flexible.

3. Ailette de ponction selon la revendication 1 ou 2, **caractérisée en ce que** le moyen de fixation est réalisé sous la forme d'un oeillet (42), à travers lequel le segment de flexible (7B) du conduit flexible (7) peut être guidé.

4. Ailette de ponction selon la revendication 3, **caractérisée en ce que** l'oeillet (42) peut être écarté afin d'insérer le segment de flexible (7B) du conduit flexible (7).

5. Ailette de ponction selon l'une quelconque des revendications 1 à 4, comprenant un conduit flexible (7) servant à amener un liquide à un patient ou servant à évacuer un liquide d'un patient, **caractérisée en ce que** le conduit flexible (7) est réalisé avec un point d'inflexion de consigne.

6. Ailette de ponction selon la revendication 5, **caractérisée en ce que** la paroi du conduit flexible (7) présente, dans la zone du point d'inflexion, une épaisseur inférieure.

7. Ailette de ponction selon la revendication 5, **caractérisée en ce que** le conduit flexible (7) présente, à l'exception de la zone du point d'inflexion de consigne, une section transversale ronde, dans laquelle le conduit flexible présente, dans la zone du point d'inflexion de consigne, une section transversale non ronde.
